# EUROPEAN PATENT APPLICATION

(11) **EP 4 825 367 A1**
(43) Date of publication of application: **21.10.2026**
(21) Application number: 24904160.9
(22) Date of filing: 04.12.2024
(51) Int. Cl.: A61K 8/44, A61K 8/60, A61Q 19/00

(54) **COSMETIC COMPOSITION FOR SKIN SOOTHING**

(30) Priority: 12.12.2023 KR 20230179923
(71) Applicant: LG H&H Co., Ltd., Seoul 04637 (KR)
(72) Inventor: JEON, Hyung Joon, Seoul 07795 (KR); KIM, Ji Yun, Seoul 07795 (KR); KIM, Mi Sun, Seoul 07795 (KR); BAEK, Woon Ki, Seoul 07795 (KR); PARK, Sang Wook, Seoul 07795 (KR)
(74) Representative: Goddar, Heinz J.
(86) International application number: PCT/KR2024/019747
(87) International publication number: WO 2025/127595

(57) **Abstract**

The present invention relates to a cosmetic composition, and provides a cosmetic composition comprising an amino acid-amino acid eutectic mixture and troxeutin as active ingredients, thus providing the effects of inhibiting inflammation and alleviating/reducing skin irritation.

## Description

### [Technical Field]

The present invention relates to a cosmetic composition for skin soothing, and more particularly, to providing a composition including, as active ingredients, an amino acid-amino acid eutectic mixture and troxerutin, which exhibits anti-inflammatory effects and alleviates/reduces skin irritation, thereby providing a hypoallergenic external skin preparation or an external skin preparation for skin soothing.

### [Background Art]

There is a growing need among cosmetics consumers for products that can effectively and sufficiently soothe sensitive or irritated skin. Furthermore, many of these consumers identify themselves as having "sensitive skin." As a result, while the market for skin-soothing cosmetics and products dedicated to sensitive skin is expanding, there remains a lack of research on effective materials for soothing sensitive and irritated skin, or on synergistic combinations thereof.

A previously developed eutectic mixture (Korean Patent Registration No. 10-2411385) exhibits excellent effects in regenerating the skin, promoting collagen synthesis, and improving skin elasticity, but it is insufficient for soothing sensitive or irritated skin.

Troxerutin, derived from the Japanese pagoda tree (*Sophora japonica*), reportedly acts as an antioxidant to scavenge free radicals and prevent oxidative stress when applied topically, and is known to protect the skin from UVB rays. However, troxerutin has a problem of insufficient skin permeability.

Against this background, the present inventors conducted extensive research to enhance the skin-soothing effect of amino acid-amino acid eutectic mixtures and improve the skin permeability of troxerutin. As a result, they discovered that using an amino acid-amino acid eutectic mixture together with troxerutin produces a synergistic effect between materials, thereby completing the present invention.

### [Disclosure]

### [Technical Problem]

An object of the present invention is to provide a cosmetic composition including, as active ingredients, an amino acid-amino acid eutectic mixture and troxerutin.

### [Technical Solution]

The present invention is explained in detail as follows. Meanwhile, each description and embodiment disclosed in the present invention may also be applied to other descriptions and embodiments. That is, all combinations of various elements disclosed in the present invention fall within the scope of the present invention. In addition, the scope of the present invention is not construed as being limited by the specific descriptions provided below.

To achieve the above object, the present invention provides a cosmetic composition including, as active ingredients, an amino acid-amino acid eutectic mixture and troxerutin.

To achieve the above object, the present invention provides a method for skin soothing or anti-inflammation, including applying a composition including, as active ingredients, an amino acid-amino acid eutectic mixture and troxerutin to the skin.

To achieve the above object, the present invention provides a method for skin soothing or anti-inflammation, including administering a composition including, as active ingredients, an amino acid-amino acid eutectic mixture and troxerutin to a subject.

To achieve the above object, the present invention provides a use of a composition including, as active ingredients, an amino acid-amino acid eutectic mixture and troxerutin for the manufacture of a cosmetic, food, or quasi-drug for skin soothing or anti-inflammation.

Hereinafter, the present invention will be described in more detail.

As used herein, the term "skin soothing" refers to alleviating irritation in sensitive skin, inhibiting the expression of inflammatory factors in the skin, and imparting a cooling or refreshing feeling to the skin.

As used herein, the term "anti-inflammatory" refers to the purpose of suppressing and preventing inflammation, which involves protecting the skin, as well as alleviating and resisting inflammation.

As used herein, the term "eutectic mixture" refers to a eutectic compound formed by a combination of polar compounds, which may include amino acids, and may be an amino acid-amino acid eutectic mixture prepared according to Korean Patent Registration No. 10-2626486.

Depending on the types of constituent amino acids, the amino acid-amino acid eutectic mixture may take the form of an amino acid-amino acid mixture or an amino acid-amino acid-one or more solvents mixture, which is determined by the characteristics of the paired amino acids.

Specifically, there must be a difference in polarity between the polar compounds, and the amino acids may be such polar compounds. In the case of polar amino acids, they may include polar, acidic, and basic amino acids. The polar amino acids may include serine, threonine, cysteine, tyrosine, asparagine, and glutamine; the acidic amino acids may include aspartic acid and glutamic acid; and the basic amino acids may include arginine, histidine, and lysine. Preferably, the eutectic mixture may be an arginine-glutamate eutectic mixture or a serine-arginine eutectic mixture.

As used herein, the term "arginine" refers to a basic amino acid with the chemical formula C₆H₁₄N₄O₂. As one of the α-amino acids, L-arginine exists as one of the amino acids that make up proteins and is abundant in the protein protamine found in fish sperm. In addition, it is a component of the ornithine cycle, an in vivo metabolic pathway, and is decomposed into urea and ornithine by the action of arginase. It is a semi-essential amino acid required for the growth of children and animals. Specifically, the arginine may be a basic amino acid.

As used herein, the term "glutamic acid" (abbreviated as Glu, E) is an acidic α-amino acid with the chemical formula C₅H₉NO₄ and a molecular weight of 147.13. Discovered early on in gluten, L-glutamic acid is widely distributed in general proteins, and is contained in large amounts particularly in cereal proteins. Specifically, the glutamic acid may be an acidic amino acid.

The total content of the amino acids in the eutectic mixture may be 1 to 100 parts by weight, 5 to 90 parts by weight, 10 to 80 parts by weight, 15 to 70 parts by weight, 20 to 65 parts by weight, 30 to 60 parts by weight, 35 to 55 parts by weight, 40 to 50 parts by weight, or 42 to 47 parts by weight, based on 100 parts by weight of the entire eutectic mixture, but is not limited thereto.

The amino acid-amino acid eutectic mixture may further include one or more solvents.

The solvent may be one or more selected from water, ethanol, methanol, and acetone, but is not limited thereto, and may be any solvent capable of dissolving amino acids.

In the present invention, the solvent may be included in an amount of 0 to 98 parts by weight based on 100 parts by weight of the entire eutectic mixture.

When the content of the solvent in the eutectic mixture is 0, a stable eutectic mixture may be formed in an anhydrous form. When the content of the solvent exceeds 98 parts by weight, hydrogen bonding stability may be compromised, preventing the formation of the eutectic mixture and causing it to revert to a general mixture form. In this case, the skin permeability of troxerutin decreases, making it difficult to achieve the desired effect.

Preferably, the eutectic mixture of the present invention may include the solvent in an amount of 10 to 90 parts by weight, 20 to 80 parts by weight, 30 to 70 parts by weight, or 40 to 60 parts by weight, based on 100 parts by weight of the entire eutectic mixture, but is not limited thereto.

The ratio of the components required to form the eutectic mixture may be determined according to the charge of the molecules constituting the eutectic mixture, the type and number of functional groups present in the molecules, and the polarity of the molecules or functional groups. The ratio of the components required for the eutectic mixture may also be determined according to the size or similarity of the molecules.

The molar concentration ratio of the respective amino acids for forming the eutectic mixture may range from 10:1 to 10:100.

Preferably, the molar concentration ratio of the respective amino acids for forming the eutectic mixture may range from 1:1 to 1:3.

The eutectic mixture according to the present invention may have its acidity adjusted to a pH of 3 to 10 in order to reduce discomfort, such as skin irritation, upon application to the skin. Structurally, the eutectic mixture of the present invention may be prepared in a pH range in which the amine groups of the amino acids are charged, and such a range may be pH 3 or higher. For example, the eutectic mixture may be adjusted to have a weakly acidic to neutral acidity, such as pH 4 to 9, pH 5 to 8, or pH 6 to 7.

By including an amino acid-amino acid combination or an amino acid-amino acid-one or more solvents combination, the eutectic mixture of the present invention may enhance the stability of the composition through the melting point depression and solubility-enhancing properties inherent to eutectic mixtures.

In general, since the skin efficacy of amino acids tends to be enhanced as pH decreases, conventional cosmetic compositions have maintained a low pH. In contrast, the eutectic mixture of the present invention may reduce skin irritation that may occur under weakly acidic to neutral conditions while increasing the skin permeability of the amino acids, thereby maintaining the high efficacy of the amino acids even at a weakly acidic to neutral pH through enhanced permeation, just as in conventional cosmetic compositions maintained at a low pH.

The cosmetic composition according to the present invention may include the eutectic mixture in an amount of 0.001 to 50 parts by weight based on the total weight of the cosmetic composition, for example, 0.01 to 50 parts by weight, 0.05 to 40 parts by weight, 0.05 to 30 parts by weight, 0.1 to 30 parts by weight, 0.1 to 25 parts by weight, 0.5 to 25 parts by weight, 0.1 to 50 parts by weight, 1 to 35 parts by weight, 5 to 30 parts by weight, 0.5 to 40 parts by weight, 1 to 30 parts by weight, 5 to 28 parts by weight, 8 to 25 parts by weight, 1 to 50 parts by weight, 5 to 40 parts by weight, 10 to 35 parts by weight, 15 to 25 parts by weight, 18 to 25 parts by weight, 15 to 20 parts by weight, 10 to 20 parts by weight, 10 to 30 parts by weight, 10 to 40 parts by weight, 15 to 35 parts by weight, 20 to 30 parts by weight, or 1 to 10 parts by weight, but is not limited thereto. The content of the eutectic mixture may be appropriately adjusted depending on the formulation of the cosmetic composition.

As used herein, the term "troxerutin" refers to a semisynthetic compound derived from rutinoside, a flavonoid originating from the Japanese pagoda tree, the compound inhibiting factors acting on leukocytes and vascular endothelial cells that are involved in the secretion of inflammatory mediators and inflammatory metabolic responses.

More specifically, troxerutin has been reported to act as an antioxidant when applied topically, scavenging free radicals and preventing oxidative stress, and is also known to protect the skin from UVB. However, troxerutin has a disadvantage in that its skin permeability is not high.

Accordingly, the present invention confirmed through specific examples that a synergistic skin-soothing and anti-inflammatory effect is achieved when an amino acid-amino acid eutectic mixture and troxerutin are included together as active ingredients.

The content of troxerutin may be 0.0001 to 2 parts by weight based on 100 parts by weight of the total composition.

When troxerutin is included in an amount of 0.0001 to 2 parts by weight based on 100 parts by weight of the total composition, the inhibitory effect on inflammatory factors or nitric oxide (NO) may be significantly enhanced.

The ratio of troxerutin to the eutectic mixture may be 1:0.1 to 1:100, preferably 1:0.2 to 1:50, and more preferably 1:1 to 1:20.

The composition may increase the skin permeability of troxerutin 1.5- to 10-fold compared with troxerutin alone.

More specifically, when the composition is applied to the skin, it may increase the skin permeability of troxerutin 1.5- to 10-fold compared to treatment with troxerutin alone. Preferably it may increase the skin permeability 2- to 10-fold.

When troxerutin is applied alone, its skin permeability may be less than 0.1%, and accordingly, the anti-inflammatory effect of the cosmetic composition may be insufficient. However, it was confirmed that the cosmetic composition of the present invention, by using troxerutin together with the eutectic mixture, increases the skin permeability of troxerutin 1.5-to 10-fold compared to using troxerutin alone, thereby enhancing skin-soothing and anti-inflammatory effects and producing a synergistic effect.

The cosmetic composition according to the present invention may further include an oil-phase component.

The oil-phase component may be one or more of an oil and a wax. As the oil and wax, any of those conventionally used as cosmetic ingredients in the art may be applied. For example, the oil may be a silicone-based oil, an ester-based oil, a triglyceride-based oil, a hydrocarbon-based oil, or a vegetable oil, and one or more of these components may be blended and used as needed.

For instance, the silicone-based oil may be a silicone-based fluid oil or a silicone-based crosspolymer dispersed in an oil. For example, the silicone-based fluid oil may include cyclopentasiloxane, cyclohexasiloxane, cycloheptasiloxane, cyclomethicone, cyclophenylmethicone, cyclotetrasiloxane, cyclotrisiloxane, dimethicone, capryl dimethicone, caprylyl trimethicone, caprylyl methicone, cetearyl methicone, hexadecyl methicone, hexyl methicone, lauryl methicone, myristyl methicone, phenyl methicone, stearyl methicone, stearyl dimethicone, trifluoropropyl methicone, cetyl dimethicone, diphenylsiloxy phenyl trimethicone, dimethyl polysiloxane, methyl phenyl polysiloxane, decamethyl cyclopentasiloxane, methyl trimethicone, or phenyl trimethicone. These silicone-based oil components may be used alone or in a combination of two or more thereof. The silicone-based crosspolymer may include a dimethicone crosspolymer, a dimethicone/vinyl dimethicone crosspolymer, a dimethicone PEG-10/15 crosspolymer, or a PEG-12 dimethicone/PPG-20 crosspolymer, but is not limited thereto.

The ester-based oil may include ascorbyl palmitate, ascorbyl linoleate, ascorbyl stearate, diisostearyl malate, benzyl benzoate, benzyl laurate, butylene glycol dicaprylate/dicaprate, butylene glycol diisononanoate, butylene glycol laurate, butylene glycol stearate, butyl isostearate, cetearyl isononanoate, cetearyl nonanoate, cetyl caprylate, cetyl ethylhexanoate, cetyl isononanoate, ethylhexyl caprylate/caprate, ethylhexyl isononanoate, ethylhexyl isostearate, ethylhexyl laurate, hexyl laurate, octyldodecyl isostearate, isopropyl isostearate, isostearyl isononanoate, isostearyl isostearate, isocetyl ethylhexanoate, neopentyl glycol dicaprate, neopentyl glycol diethylhexanoate, neopentyl glycol diisononanoate, neopentyl glycol diisostearate, pentaerythrityl stearate, pentaerythrityl tetraethylhexanoate, Dipentaerythrityl hexa acid ester, polyglyceryl-2 diisostearate, polyglyceryl-2 sesquiisostearate, polyglyceryl-2 isostearate, polyglyceryl-2 tetraisostearate, polyglyceryl-2 triisostearate, polyglyceryl-3 diisostearate, polyglyceryl-3 isostearate, polyglyceryl-4 diisostearate, polyglyceryl-4 isostearate, polyglyceryl-6 diisostearate, polyglyceryl-6 sesquiisostearate, or triethylhexanoin.

The triglyceride-based oil may include C8-C12 acid triglycerides, C12-C18 acid triglycerides, caprylic/capric triglycerides, caprylic/capric/lauric triglycerides, C10-C40 isoalkyl acid triglycerides, C10-C18 triglycerides, glyceryl triacetyl hydroxystearate, soybean glycerides, tribehenin, tricaprin, triethylhexanoin, triheptanoin, triisostearin, tripalmitin, or tristearin.

The hydrocarbon-based oil may include liquid paraffin (mineral oil), paraffin, Vaseline, microcrystalline wax, or squalane.

The vegetable oil may include avocado oil, wheat germ oil, rosehip oil, shea butter, almond oil, olive oil, macadamia oil, argan oil, meadowfoam oil, sunflower oil, castor oil, camellia oil, corn oil, safflower oil, soybean oil, rapeseed oil, macadamia nut oil, jojoba oil, palm oil, palm kernel oil, or coconut oil.

As the wax, any wax generally used in cosmetics, such as a hydrocarbon-based wax, a vegetable wax, or a silicone-based wax, may be used. Examples thereof may include, but are not limited to, candelilla wax, carnauba wax, rice wax, beeswax, lanolin, ozokerite, ceresin wax, paraffin wax, microcrystalline wax, C30-45 alkyl dimethyl silyl polypropylsilsesquioxane, ethylene/propylene copolymers, or polyethylene wax.

The content of the oil-phase component is not particularly limited, and the oil-phase component may be included as the remaining balance of the composition excluding the aforementioned eutectic mixture.

The cosmetic composition may be for skin soothing.

The cosmetic composition of the present invention may further include all types of ingredients usable in conventional cosmetics, without limitation, for example, moisturizers such as glycerin, butylene glycol, propylene glycol, hexanediol, methyl gluceth-20, diglycerin, and ethylhexylglycerin; UV blockers such as ethylhexyl methoxycinnamate, ethylhexyl salicylate, ethylhexyl triazone, octocrylene, and bis-ethylhexyloxyphenol methoxyphenyl triazine; pH adjusters such as triethanolamine; thickeners such as carbomer, xanthan gum, acrylates/C10-30 alkyl acrylate crosspolymer, and hyaluronic acid; preservatives such as phenoxyethanol, methylparaben, and propylparaben; antioxidants such as BHT, ethyl ascorbyl ether, and ascorbic acid; skin conditioning agents such as beta-glucan; surfactants such as cetearyl glucoside and sorbitan stearate; and fragrances or colorants.

Each of the above ingredients included in the cosmetic composition according to the present invention is preferably included in an amount that does not exceed the maximum usage limits prescribed by the "Safety and Technical Standards for Cosmetics" designated by the Chinese government.

The cosmetic composition according to the present invention may be formulated into any formulation conventionally prepared in the art. For example, the cosmetic composition may have a formulation such as, but not limited to, skin softeners, nutrient toners, spray-type toners, emulsions such as facial lotions and body lotions, creams such as nutrient creams, moisture creams, and eye creams, sticks, essences, cosmetic ointments, sprays, gels, packs, sunscreens, makeup bases, liquid or spray-type foundations, powders, makeup removers such as cleansing lotions and cleansing oils, and cleansers such as cleansing foams, soaps, and body washes.

In one embodiment, the formulation of the cosmetic composition may be a toner/skin solubilization formulation, an oil-in-water (O/W) emulsion formulation, an O/W cream formulation, or a water-in-silicone (W/S) cream formulation.

As used herein, the "solubilization formulation" refers to a formulation in which a small amount of an oil component is transparently dissolved in water, and which exhibits a transparent phase because the diameter of the oil drops is smaller than the wavelength of visible light, thereby not interfering with the straightness of light without scattering or reflection. The cosmetic composition having the solubilization formulation may be a transparent skin lotion, toner, hair tonic, or hair liquid, but the type thereof is not limited thereto.

The cosmetic composition of the present invention may be used according to a conventional method of use, and the frequency of use may vary depending on the user's skin condition or preference.

To achieve the above object, another aspect of the present invention provides an anti-inflammatory composition including an amino acid-amino acid eutectic mixture and troxerutin as active ingredients.

In a specific example, it was confirmed that the cosmetic composition of the present invention can provide a cosmetic composition having high skin-soothing and anti-inflammatory effects by increasing the skin permeability of troxerutin compared to when troxerutin is used alone, thereby solving the problem of low skin permeability of troxerutin.

The advantages and features of the present invention, and methods of achieving them, will become apparent with reference to Experimental Examples and Preparation Examples described in detail below. However, the present invention is not limited to Experimental Examples and Preparation Examples disclosed below, but may be embodied in various different forms. Rather, these examples are provided so that this disclosure will be thorough and complete, and will fully convey the scope of the invention to those of ordinary skill in the art.

### [Advantageous Effects]

The cosmetic composition for skin soothing and anti-inflammation according to the present invention includes a eutectic mixture and troxerutin, exhibiting effects of increasing the permeability of troxerutin, inhibiting inflammation, and alleviating skin irritation, thereby being provided as a hypoallergenic external skin preparation or an external skin preparation for skin soothing.

### [Best Mode]

Hereinafter, the present invention will be described in more detail through Examples. These Examples are provided only for illustrating the present invention in more detail, and the scope of the present invention is not limited thereby.

### Example 1: Evaluation of NO Expression Inhibitory Effect

To confirm the anti-inflammatory effect of the cosmetic composition of the present invention, the nitric oxide (NO) production inhibitory effect of each prepared sample was evaluated using RAW 264.7 cells.

Specifically, RAW 264.7 cells were pre-treated with each sample as shown in Table 1 below for 30 minutes, and then 500 ng/mL of lipopolysaccharide (LPS) was added, followed by incubation for 18 hours. After incubation, the supernatant was collected, transferred to a 96-well plate, and allowed to react with GRIESS reagent at room temperature for 15 minutes. The absorbance at 540 nm was measured using an ELISA reader, and the NO production inhibitory ability was calculated using the following equation. The results are shown in Table 1. 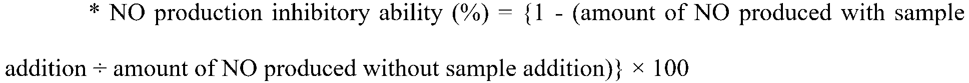 * NO production inhibitory ability (%) = {1 - (amount of NO produced with sample addition ÷ amount of NO produced without sample addition)} × 100
* Positive control: 100 µM L-NMMA (80.9% NO production inhibition rate)
* Negative control: RAW 264.7 cells treated with LPS only

**[Table 1]**

| | NO Production Inhibitory Ability (%) |
|---|---|
| Arginine-glutamate eutectic mixture 10 ppm | 0 |
| Arginine-glutamate eutectic mixture 100 ppm | 0 |
| Troxerutin 1 ppm | 0 |
| Troxerutin 1 ppm + Arginine-glutamate eutectic mixture 0.1 ppm | 14.0 |
| Troxerutin 1 ppm + Arginine-glutamate eutectic mixture 10 ppm | 18.4 |
| Troxerutin 1 ppm + Arginine-glutamate eutectic mixture 100 ppm | 25.3 |
| Positive control (L-NMMA) 100 µM | 80.9 |

As shown in Table 1, treatment with troxerutin or the arginine-glutamate eutectic mixture alone did not exhibit an inhibitory effect on the production of the inflammatory factor NO. In contrast, the combination of the "troxerutin + arginine-glutamate eutectic mixture" according to the present invention demonstrated an NO production inhibitory ability, thereby confirming the anti-inflammatory effect of the cosmetic composition of the present invention.

### Example 2: Evaluation of Cytokine IL-1β Expression Inhibitory Effect

To confirm the anti-inflammatory effect of the combination of troxerutin and the arginine-glutamate eutectic mixture, the inhibitory effect on the production of a sensitive irritation factor (cytokine) induced by capsaicin was evaluated in a human keratinocyte cell line.

Specifically, human keratinocytes were pre-treated with each sample as shown in Table 2 below for 1 hour, and then 50 µm capsaicin was added, followed by incubation for 24 hours. After incubation, RNA was extracted and cDNA was synthesized, followed by real-time polymerase chain reaction (real-time PCR) to measure the amount of IL-1β. The sensitive inflammatory cytokine (IL-1β) production inhibitory ability was evaluated using the following equation, and the results are shown in Table 2. 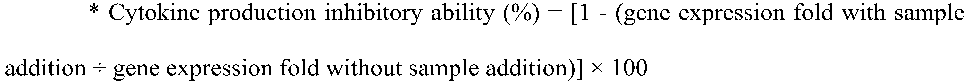 * Cytokine production inhibitory ability (%) = [1 - (gene expression fold with sample addition ÷ gene expression fold without sample addition)] × 100

* Negative control: Human keratinocyte cell line treated with capsaicin only

**[Table 2]**

| | IL-1β Inhibition Rate (%) |
|---|---|
| Arginine-glutamate eutectic mixture 10 ppm | -0.73 |
| Troxerutin 1 ppm | 25.38 |
| Troxerutin 1 ppm + Arginine-glutamate eutectic mixture 10 ppm | 55.6 |

As shown in Table 2, the arginine-glutamate eutectic mixture alone rather increased the expression of the inflammatory factor IL-1β. However, compared to the IL-1β expression inhibition rate of troxerutin alone, the combination of the troxerutin + arginine-glutamate eutectic mixture of the present invention exhibited a more than 2-fold greater inhibitory effect on the inflammatory factor. Thus, it was confirmed that a synergistic effect is exhibited by using the arginine-glutamate eutectic mixture together with troxerutin.

### Example 3: Evaluation of Skin Permeability

The skin permeability of the combination of troxerutin and the arginine-glutamate eutectic mixture was evaluated.

Specifically, the amount of skin adsorption was evaluated using a Franz Cell (FCDV-15, Labfine). A transdermal diffusion testing membrane (Start-M 25 mm, Merck), which can predict skin permeability, was positioned between a donor chamber and a receptor chamber, and 3 mL of PBS solution was added to the receptor chamber.

As the evaluation method, 100 µL of each of [1% troxerutin] and [1% troxerutin + 10% arginine-glutamate eutectic mixture] was applied onto the membrane. Subsequently, rolling was performed 10 times for 30 seconds using a polyethylene stick, and permeation was then allowed to proceed in a constant temperature and humidity chamber at 37.5 °C and 50% relative humidity for 24 hours. Thereafter, the content of troxerutin in the receptor chamber was measured.

The concentration of troxerutin was determined by fluorescence spectrum measurement (Ex: 360 nm, measurement range: 380 to 700 nm). After quantifying the concentration based on the fluorescence intensity measured at 470 nm, the permeated amount of troxerutin was calculated using the resulting value and the volume of the extraction solvent. The results are shown in Table 3 below.

**[Table 3]**

| | Troxerutin Permeation Amount | Permeability |
|---|---|---|
| Troxerutin 1% | 0.86 µg | 0.086% |
| Troxerutin 1% + Arginine-glutamate eutectic mixture 10% | 2.06 µg | 0.206% |

As shown in Table 3, it was confirmed that the permeability of troxerutin increased by about 2.4-fold in the combination of troxerutin + arginine-glutamate eutectic mixture compared to troxerutin alone. Therefore, it was confirmed that the conventionally known problem of the low skin permeability of troxerutin is resolved by the present invention.

Summarizing Examples 1 to 3 of the present invention, it was confirmed that the cosmetic composition of the present invention exhibits anti-inflammatory effects and alleviates/reduces skin irritation by being provided as a hypoallergenic external skin preparation or an external skin preparation for skin soothing.

From the above description, those skilled in the art to which the present invention pertains will understand that the present invention can be embodied in other specific forms without changing the technical spirit or essential characteristics thereof. In this regard, it should be understood that the examples described above are illustrative in all respects and not restrictive. The scope of the present invention should be construed as including all changes or modified forms derived from the meaning and scope of the appended claims and their equivalent concepts, rather than the detailed description above, fall within the scope of the present invention.

## Claims

1. A cosmetic composition comprising, as active ingredients:
an amino acid-amino acid eutectic mixture; and
troxerutin.

2. The cosmetic composition of claim 1, wherein the content of the amino acid-amino acid eutectic mixture is 0.001 to 50 parts by weight based on 100 parts by weight of the total composition.

3. The cosmetic composition of claim 1, wherein the amino acid-amino acid eutectic mixture includes an arginine-glutamate eutectic mixture or a serine-arginine eutectic mixture.

4. The cosmetic composition of claim 1, wherein the content of troxerutin is 0.0001 to 2 parts by weight based on 100 parts by weight of the total composition.

5. The cosmetic composition of claim 1, wherein the composition increases the skin permeability of troxerutin 1.5- to 10-fold compared to treatment with troxerutin alone.

6. The cosmetic composition according to any one of claims 1 to 5, wherein the cosmetic composition is for soothing the skin.

7. An anti-inflammatory composition comprising, as active ingredients:
an amino acid-amino acid eutectic mixture; and troxerutin.
